(19) Europäisches Patentamt
European Patent Office
Office européen des brevets

(11) **EP 3 967 680 A1**

(12) **EUROPEAN PATENT APPLICATION**
published in accordance with Art. 153(4) EPC

(43) Date of publication:
**16.03.2022 Bulletin 2022/11**

(21) Application number: **20918144.5**

(22) Date of filing: **13.10.2020**

(51) Int Cl.:
**C07C 403/24** *(2006.01)* **A61K 31/122** *(2006.01)*
**A61P 39/06** *(2006.01)* **A23L 33/105** *(2016.01)*
**A23L 33/145** *(2016.01)*

(86) International application number:
**PCT/CN2020/120622**

(87) International publication number:
**WO 2022/021612 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.07.2020 CN 202010733602**

(71) Applicant: **Ocean University of China
Qingdao, Shandong 266100 (CN)**

(72) Inventors:
• **QI, Xiangming**
  **Qingdao, Shandong 266100 (CN)**
• **LIANG, Kanglin**
  **Qingdao, Shandong 266100 (CN)**
• **HUANG, Wencan**
  **Qingdao, Shandong 266100 (CN)**
• **MAO, Xiangzhao**
  **Qingdao, Shandong 266100 (CN)**

(74) Representative: **Cesa, Roberta
Buzzi, Notaro & Antonielli d'Oulx S.p.A.
Corso Vittorio Emanuele II, 6
10123 Torino (IT)**

(54) **METHOD FOR PREPARING WATER-SOLUBLE ASTAXANTHIN, AND ASTAXANTHIN AQUEOUS SOLUTION PREPARED USING SAID METHOD**

(57) Provided is a method for preparing water-soluble astaxanthin complex. The method includes mixing a raw material containing astaxanthin with a solution containing an organic acid and performing cell disruption and leaching and can enable natural astaxanthin to interact with components such as proteins, nucleic acids, or polysaccharides that naturally exist in the raw material, so as to directly prepare water-soluble astaxanthin complex without modifying astaxanthin. The method requires a natural source of astaxanthin, has low equipment requirements and production costs, is simple to operate, green and safe with no organic solvent residues, and easy to industrialize.

Fig. 1

Printed by Jouve, 75001 PARIS (FR)

**Description**

TECHNICAL FIELD

The present application relates to the technical field of biochemical engineering and, in particular, to a method for preparing water-soluble astaxanthin complex and an aqueous solution of astaxanthin prepared thereby.

BACKGROUND

Astaxanthin has physiological activities such as protecting skins from light damages, preventing arteriosclerosis and related diseases, anti-cancer activity, enhancing the functions of the immune system, maintaining the health of eyes and central nervous systems and anti-infection, and is widely applied to the fields of foods, medicine, cosmetics, and breeding industry. Therefore, the related extraction and application technologies of astaxanthin are attracting more and more attention.

In current researches of patents and related documents, the technologies for extracting astaxanthin mainly include organic solvent extraction, supercritical $CO_2$ extraction, enzymatic hydrolysis, and microwave extraction. However, there are some problems with these methods.

CN110724083A has disclosed an organic solvent extraction method. During extraction, due to the unstable structure of astaxanthin, an isomerization reaction may occur so that the antioxidant activity of astaxanthin is reduced. In addition, most organic solvents used in this type of organic solvent extraction method have certain toxicity, and the problem of reagent residues after the extraction limits the application of products in the food industry.

CN109608376A has disclosed a supercritical $CO_2$ extraction method which requires an ability to withstand dozens of atmospheres in the production and application, high equipment requirements, large capital investment, and high production costs. In addition, the technology involves the operations of high-pressure resistant equipment and has high requirements on production technologies, which also limits its application in industrial production.

CN107805215B has disclosed an extraction method of astaxanthin by enzymatic hydrolysis which is used for breaking the walls of Haematococcus pluvialis for a second time in an extraction process so that the extraction rate of astaxanthin is improved. However, the reaction has relatively high requirements on operation conditions such as temperature, pH, and time, the operation process is complicated, and the use of enzyme preparations also increases production costs.

In 2007, Wang Lingzhao et al. performed an organic extraction of astaxanthin with an auxiliary microwave method (see "STUDY ON THE TECHNOLOGY OF EXTRACTING ASTAXANTHIN FROM HAEMATOCOCCUS PLUVIALIS BY MICROWAVE METHOD", Wang Lingzhao et al., Food Research and Development, Volume 28, No. 12, Pages 96-100). Although the extraction rate has been improved, an increase in temperature during the microwave extraction process results in an oxidative decomposition of astaxanthin. In addition, the microwave extraction has relatively poor selectivity, which is not conducive to the separation of astaxanthin.

The above technologies and methods involve only the extraction of astaxanthin. However, astaxanthin faces many problems in product applications due to its insolubility in water regardless of many physiological activities. Therefore, the extracted astaxanthin often needs to be reprocessed to form an end product. Therefore, it has become a bottleneck problem in the process of astaxanthin productization to improve the dispersibility of astaxanthin in an aqueous solution and the stability of astaxanthin.

At present, domestic and foreign patents and researches related to the improvement in the water solubility of astaxanthin mainly include a microcapsule method, an emulsifier method, a nanoprecipitation method, an inclusion method, and the like.

CN108403666A has disclosed a microcapsule method which can improve the stability and physical properties of biomolecules. However, there are problems such as discontinuous operations which are not conducive to linked production and the difficulty in recycling embeddings which results in relatively high preparation costs.

In 2013, Navideh A et al. improved the water solubility of astaxanthin by adding hydrophilic emulsifiers (polysorbate and sucrose ester). However, this technology relies on appropriate emulsification and homogenization means and drying technology. Moreover, residual organic solvents after emulsification and volatilization also cause certain biosafety problems for astaxanthin products (see "Effects of Selected Polysorbate and Sucrose Ester Emulsifiers on the Physicochemical Properties of Astaxanthin Nanodispersions", Navideh A, et al, Molecules, No. 18, pages 768-777).

In 2007, Xiaolin Chen et al. included astaxanthin in β-cyclodextrin by an inclusion method (see "The preparation and stability of the inclusion complex of astaxanthin with β-cyclodextrin", Xiaolin Chen et al., Food Chemistry, No. 101, pages 1580-1584). Although the solubility and stability of astaxanthin are improved, the method is low in drug loading content which is generally only 9% to 14%, which causes the waste of resources, increases production costs, and is not conducive to industrial production.

CN109646425A has disclosed a method for constructing an astaxanthin nanosystem with chitosan and DNA.

The method is simple to operate and does not impose high requirements on equipment. However, the components of a polymer material are mostly obtained by chemical methods such as artificial synthesis so that the polymer material has some deficiencies in biocompatibility, degradability, cytotoxicity, and organic solvent residues.

In summary, the existing preparation process of natural astaxanthin products tends to include two steps: an extraction technology and a water-soluble astaxanthin complex preparation technology, and various technologies involved in the two-step process all have certain deficiencies.

Therefore, it is necessary to develop a method for preparing water-soluble astaxanthin complex that can alleviate the above problems, so as to improve the water solubility and stability of astaxanthin.

SUMMARY

In view of the problem in the existing art, the present application provides a method for preparing water-soluble astaxanthin complex. The method overcomes the technical deficiencies in two steps of an extraction technology and a water-soluble astaxanthin complex preparation technology, includes mixing a raw material containing astaxanthin with a solution containing an organic acid and performing cell disruption and leaching and can enable natural astaxanthin to interact with components such as proteins, nucleic acids, or polysaccharides that naturally exist in the raw material, so as to extract natural astaxanthin and prepare water-soluble astaxanthin complex in one step without separately modifying astaxanthin. The method requires a natural source of astaxanthin, has low equipment requirements and production costs, is simple to operate, green and safe with no organic solvent residues, and easy to industrialize.

To achieve this object, the present application adopts technical solutions described below.

In a first aspect, the present application provides a method for preparing water-soluble astaxanthin complex. The method includes the following steps:

(1) mixing a raw material containing astaxanthin with a solution containing an organic acid and performing cell disruption to obtain a leachate; and

(2) stirring and leaching the leachate in step (1) and performing solid-liquid separation to obtain water-soluble astaxanthin complex.

The method for preparing water-soluble astaxanthin complex provided by the present application includes mixing a raw material containing astaxanthin with a solution containing an organic acid and performing cell disruption. The interaction between astaxanthin and components such as proteins, nucleic acids, or polysaccharides that naturally exist in the raw material containing astaxanthin is promoted through the environment of the organic acid so that water-soluble astaxanthin complex in a self-assembly form is formed. These natural components are wrapped outside the astaxanthin and make the astaxanthin present stable water solubility and the astaxanthin in the prepared aqueous solution of astaxanthin will not agglomerate. In addition, the method requires a natural source of astaxanthin, has low equipment requirements and production costs, is simple to operate, green and safe with no organic solvent residues, and easy to industrialize.

In addition, the present application adopts an edible food-grade organic acid and the finally-obtained astaxanthin has no solvent residues, which can effectively ensure the chemical safety of astaxanthin in the fields of foods, biomedicine, or cosmetics.

Preferably, the raw material containing astaxanthin includes any one or a combination of at least two of Haematococcus pluvialis, Phaffia rhodozyma, crustaceans, or chlorella. Typical but non-limiting combinations are a combination of Haematococcus pluvialis and Phaffia rhodozyma, a combination of Haematococcus pluvialis and chlorella, and a combination of Phaffia rhodozyma and chlorella. Preferably, the raw material containing astaxanthin is Haematococcus pluvialis.

The raw material containing astaxanthin in the present application is preferably selected from wet algae mud of Haematococcus pluvialis.

Preferably, the organic acid in the solution containing an organic acid in step (1) has a mass concentration of 0.1 to 2.0 moL/L which may be, for example, 0.1 moL/L, 0.2 moL/L, 0.5 moL/L, 0.8 moL/L, 1 moL/L, 1.2 moL/L, 1.3 moL/L, 1.5 moL/L, 1.8 moL/L, 2moL/L, or the like.

Preferably, the solution containing an organic acid has a pH of 3.0 to 6.5 which may be, for example, 3.0, 3.2, 3.5, 3.8, 4.0, 4.2, 4.5, 4.8, 5.0, 5.2, 5.5, 5.8, 6.0, 6.2, 6.3, 6.5, or the like. Preferably, the pH is 5.0 to 6.3.

The pH of the solution containing an organic acid in the present application is preferably 5.0 to 6.3, which can better promote the interaction between astaxanthin and other natural components in the solution and increase a leaching rate.

Preferably, the organic acid includes any one or a combination of at least two of malic acid, tartaric acid, glycine, oxalic acid, or citric acid. Typical but non-limiting combinations are a combination of malic acid and tartaric acid, a combination of malic acid and glycine, a combination of malic acid and oxalic acid, a combination of tartaric acid and glycine, and a combination of glycine and citric acid. Preferably, the organic acid is citric acid.

Preferably, the solution containing an organic acid is a pH buffer solution of an organic acid.

The solution containing an organic acid in the present application is preferably a pH buffer solution of an organic acid. This is because the pH has a relatively great effect on the isoelectric points of various substances in the leachate, which will affect the degree of self-assembly between astaxanthin and natural substances in the solution, and finally affect the extraction rate and antioxidant property of astaxanthin. Moreover, the cell sap in a cell originally has a certain pH. The use of the pH buffer solution can effectively ensure the stability of the pH of the solution in a long-term leaching process and further improve the leaching rate.

Preferably, the pH buffer solution of an organic acid includes any one or a combination of at least two of a buffer solution of malic acid and sodium malate, a buffer solution of tartaric acid and sodium tartrate, a buffer solution of glycine and HCl, a buffer solution of oxalic acid and sodium oxalate, or a buffer solution of citric acid and sodium citrate. Typical but non-limiting combinations are a combination of the buffer solution of malic acid and sodium malate and the buffer solution of glycine and HCl, a combination of the buffer solution of oxalic acid and sodium oxalate and the buffer solution of citric acid and sodium citrate, a combination of the buffer solution of citric acid and sodium citrate and the buffer solution of malic acid and sodium malate, a combination of the buffer solution of oxalic acid and sodium oxalate and the buffer solution of glycine and HCl, and the combination of the buffer solution of malic acid and sodium malate and the buffer solution of glycine and HCl. Preferably, the pH buffer solution of the organic acid is a buffer solution of citric acid and sodium citrate.

Preferably, a material-to-liquid ratio of the raw material containing astaxanthin to the solution containing an organic acid is 0.1-10 g: 10-40 mL and may be, for example, 0.1 g: 10 mL, 2 g: 10 mL, 5 g: 10 mL, 10 g: 10 mL, 0.1 g: 20 mL, 0.5 g: 20 mL, 1 g: 20 mL, 5 g: 20 mL, 10 g: 20 mL, 0.1 g: 30 mL, 0.5 g: 30 mL, 1 g: 30 mL, 2 g: 30 mL, 5 g: 30 mL, 10 g: 30 mL, 0.1 g: 40 mL, 0.5 g: 40 mL, 1 g: 40 mL, 2 g: 40 mL, 5 g: 40 mL, 10 g: 40 mL or the like.

Preferably, the manner of the cell disruption in step (1) includes cell disruption by use of an ultrasonic disruptor.

Preferably, the cell disruption is performed for 30 to 60 min which may be, for example, 30 min, 35 min, 40 min, 45 min, 50 min, 55 min, 60 min, or the like.

Preferably, in step (1) and/or step (2), an auxiliary agent is added to assist in leaching.

The present application can achieve the interaction between natural astaxanthin and the components such as proteins, nucleic acids, or polysaccharides that naturally exist in the raw material by adding an auxiliary agent allowable in foods, thereby promoting the assembly of water-soluble astaxanthin complex in one step and improving the extraction rate of water-soluble astaxanthin complex.

Preferably, the auxiliary agent includes any one or a combination of at least two of proteins, nucleic acids, or polysaccharides. Typical but non-limiting combinations are a combination of proteins and nucleic acids, a combination of proteins and polysaccharides, and a combination of nucleic acids and polysaccharides.

Preferably, the stirring and leaching in step (2) is performed for 6 to 72 h which may be, for example, 6 h, 8 h, 10 h, 12 h, 15 h, 20 h, 24 h, 25 h, 30 h, 35 h, 40 h, 45 h, 48 h, 50 h, 60 h, 72 h or the like and preferably, 12 to 48 h.

Preferably, the stirring and leaching is performed at a temperature of 20 to 35 °C which may be, for example, 20 °C, 22 °C, 25 °C, 26 °C, 27 °C, 28 °C, 29 °C, 30 °C, 32 °C, 33 °C, 35 °C, or the like.

Preferably, the stirring and leaching is performed under a closed condition without light.

Preferably, the solid-liquid separation in step (2) includes centrifugal separation.

Preferably, the centrifugal separation is performed at a temperature of 2 to 5 °C which may be, for example, 2 °C, 3 °C, 4 °C, 5 °C, or the like.

Preferably, the centrifugal separation is performed for 30 to 60 min which may be, for example, 30 min, 35 min, 40 min, 45 min, 50 min, 55 min, 60 min, or the like.

Preferably, the centrifugal separation is performed at a rotational speed of 7000 to 9000 r/min which may be, for example, 7000 r/min, 7500 r/min, 7800 r/min, 8000 r/min, 8200 r/min, 8500 r/min, 8600 r/min, 9000 r/min, or the like.

Preferably, after the centrifugal separation, a supernatant obtained through the centrifugal separation is filtered with a filter to obtain a solution of water-soluble astaxanthin complex.

Preferably, the method further includes: (3) re-leaching raw material residues containing astaxanthin obtained through the solid-liquid separation in step (2) at least once.

In the present application, the leaching environment is relatively mild and it is impossible to extract astaxanthin completely once. The raw material residues containing astaxanthin after the solid-liquid separation are added with a solution containing an organic acid for another extraction, which may be repeated many times to improve the overall yield of the process.

Preferably, the re-leaching includes mixing the raw material residues containing astaxanthin with a solution containing an organic acid to obtain a leachate and repeating step (2).

Preferably, the re-leaching adopts the same solution containing an organic acid as step (1) and/or a different solution containing an organic acid from step (1).

Preferably, the re-leaching adopts the same solution containing an organic acid as step (1) and then a different solution containing an organic acid from step (1).

In the present application, the same solution containing an organic acid is preferably adopted for leaching. When the extraction rate of astaxanthin is low, the concentrations of various substances in the solution have reached the isoelectric points under this pH condition. The solution is then replaced with a solution containing an organic acid with a different pH for another extraction, which can further increase the extraction rate.

As a preferred solution of the present application, the method includes the following steps:

(1) mixing a raw material containing astaxanthin with a solution containing an organic acid and performing cell disruption to obtain a leachate, where the organic acid has a mass concentration of 0.1 to 2.0 moL/L, the solution containing an organic acid is a pH buffer solution of an organic acid with a pH of 3.0 to 6.5, and a material-to-liquid ratio of the raw material containing astaxanthin to the solution containing an organic acid is 0.1-10 g: 10-40 mL;

(2) stirring and leaching the leachate in step (1) for 6 to 72 h at 20 to 35 °C under a closed condition without light, performing centrifugal separation for 30 to 60 min at 2 to 5 °C and 7000 to 9000 r/min, and filtering a supernatant obtained through the centrifugal separation with a filter to obtain water-soluble astaxanthin complex; and

(3) mixing raw material residues containing astaxanthin obtained through solid-liquid separation in step (2) with a solution containing an organic acid to obtain a leachate and repeating step (2), where the re-leaching is performed at least once.

In a second aspect, the present application provides an aqueous solution of astaxanthin prepared by the method for preparing water-soluble astaxanthin complex described in the first aspect.

The aqueous solution of astaxanthin provided by the present application contains a relatively high content of astaxanthin and astaxanthin is stable and not polymerized and can be stored well.

In a third aspect, the present application provides a lyophillized powder of astaxanthin prepared by lyophillizing the aqueous solution of astaxanthin described in the second aspect.

The present application may also lyophillize the aqueous solution of astaxanthin to obtain the lyophillized powder of astaxanthin which can not only increase the concentration of astaxanthin but also has good resolubility when dissolved in water again.

Compared with the existing art, the present application has beneficial effects described below.

(1) The method for preparing water-soluble astaxanthin complex provided by the present application overcomes the deficiencies of two steps of astaxanthin extraction and water-soluble astaxanthin complex preparation which are coupled to obtain water-soluble astaxanthin complex in one step. The concentration of astaxanthin in the prepared aqueous solution of astaxanthin can reach 72.66 μg/mL or more, the first extraction rate can reach 37.45wt% or more at most, and the extraction rate of multiple extractions can reach 68.05wt% or more.

(2) The method for preparing water-soluble astaxanthin complex provided by the present application adopts a solution containing an organic acid safe for use in foods, has no solvent residues, and is mild to operate and suitable for industrial production.

(3) The aqueous solution of astaxanthin provided by the present application has stable properties, a high content of astaxanthin, and good resolubility after lyophillized.

BRIEF DESCRIPTION OF DRAWINGS

FIG. 1 is a flowchart of a method for preparing water-soluble astaxanthin complex according to the present application.

FIG. 2 is a diagram of water-soluble astaxanthin complex prepared in Example 1 of the present application under a transmission electron microscope.

DETAILED DESCRIPTION

The technical solutions of the present application are further described below through embodiments in conjunction with drawings.

The present application is further described in detail below. The examples described below are merely simple examples of the present application and not intended to represent or limit the scope of the present application. The protection scope of the present application is defined by the claims.

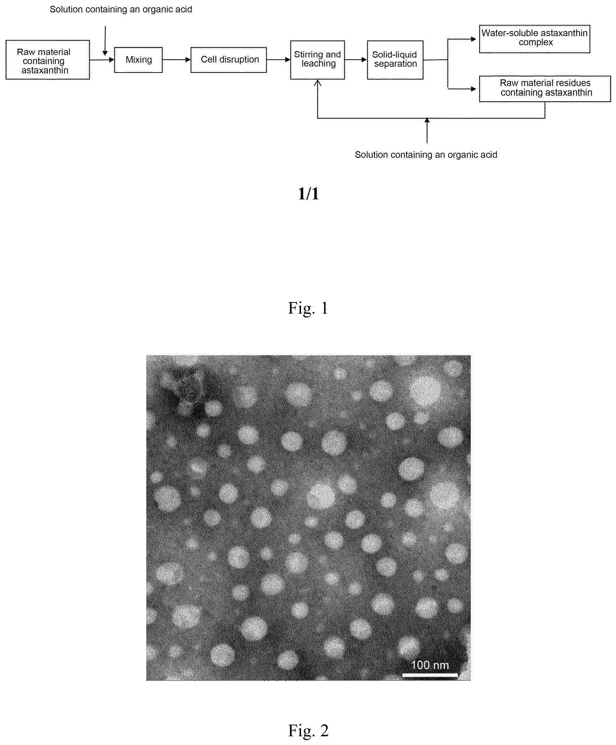

As shown in FIG. 1 which is a flowchart of a method for preparing water-soluble astaxanthin complex according to the present application, the method specifically includes the following steps:

(1) mixing a raw material containing astaxanthin with a solution containing an organic acid and performing cell disruption to obtain a leachate;

(2) stirring and leaching the leachate in step (1) and performing solid-liquid separation to obtain water-soluble astaxanthin complex; and

(3) mixing raw material residues containing astaxanthin obtained through the solid-liquid separation in step (2) with the solution containing an organic acid to obtain a leachate and repeating step (2), where re-leaching is performed at least once.

I. Examples

Example 1

This example provides a method for preparing water-soluble astaxanthin complex. The method includes the following steps:

(1) Haematococcus pluvialis (wet algae) was taken out from a refrigerator of -20 °C and thawed, 1 g of Haematococcus pluvialis algae mud was weighed and added with 20 mL of a buffer solution of citric acid/sodium citrate with a concentration of 0.1 mol/L and a pH of 6.0, they were mixed thoroughly, and the mixed solution of Haematococcus pluvialis was disrupted for 30 min with an ultrasonic disruptor to obtain a leachate;

(2) the leachate in step (1) was transferred to a 50 mL brown finger-shaped bottle, placed on a magnetic stirrer, and stirred and leached at 25 °C for 24 h in a closed environment without light; and

(3) the mixed solution after leaching in step (2) was transferred to a centrifuge tube and centrifuged at 4 °C and 8000 rpm for 30 min with a centrifuge, and a supernatant after centrifugation was filtered with a disposable filter with a pore size of 3 $\mu$m to obtain water-soluble astaxanthin complex that was uniform, stable, orange-red and translucent and had a concentration of 72.66 $\mu$g/mL.

The morphological characterization of water-soluble astaxanthin complex prepared in this example was carried out. A drop of freshly prepared water-soluble astaxanthin complex was dropped on a clean paraffin board and a copper mesh was gently placed on the surface of the drop so that the drop was immersed in the copper mesh. The copper mesh was removed after 10 min, the liquid on the surface of the copper mesh was wiped off with a filter paper, and the copper mesh was then placed with the surface with the liquid up. The front face of the copper mesh was dyed with 1% phosphotungstic acid for 10 min, dried, and observed with a transmission electron microscope (JEM-1400 transmission electron microscope produced by Japan JEOL) whose field of view was adjusted.

The diagram of water-soluble astaxanthin complex prepared in this example under the transmission electron microscope is shown in FIG. 2. The prepared water-soluble astaxanthin complex has a regular and complete shape and a spherical shape with good dispersibility, and most astaxanthin particles have a diameter within a range of 40 to 100 nm, do not agglomerate, and have relatively high stability.

Example 2

This example provides a method for preparing water-soluble astaxanthin complex, which is the same as that in Example 1 except that "a buffer solution of citric acid/sodium citrate with a pH of 6.0" in step (1) was replaced with "a buffer solution of citric acid/sodium citrate with a pH of 3.0".

Example 3

This example provides a method for preparing water-soluble astaxanthin complex, which is the same as that in Example 1 except that "a buffer solution of citric acid/sodium citrate with a pH of 6.0" in step (1) was replaced with "a buffer solution of citric acid/sodium citrate with a pH of 4.0".

Example 4

This example provides a method for preparing water-soluble astaxanthin complex, which is the same as that in Example 1 except that "a buffer solution of citric acid/sodium citrate with a pH of 6.0" in step (1) was replaced with "a buffer solution of citric acid/sodium citrate with a pH of 5.0".

Example 5

This example provides a method for preparing water-soluble astaxanthin complex, which is the same as that in Example 1 except that "a buffer solution of citric acid/sodium citrate" in step (1) was replaced with "a buffer solution of glycine/HCl".

Example 6

This example provides a method for preparing water-soluble astaxanthin complex. The method performs re-extraction six times on raw material residues containing astaxanthin obtained through the centrifugal separation in step (3) in Example 1, which specifically includes the following steps:

the raw material residues containing astaxanthin obtained through the centrifugal separation in step (3) were mixed with a buffer solution of citric acid/sodium citrate with a concentration of 0.1 mol/L and a pH of 6.0 at a material-to-liquid ratio of 1 g : 20 mL to obtain a leachate, step (2) was repeated, and the re-leaching was repeated six times.

Example 7

This example provides a method for preparing water-soluble astaxanthin complex. The method performs re-extraction 10 times on raw material residues containing astaxanthin obtained through the centrifugal separation in step (3) in Example 1, which specifically includes the following steps:

(4) the raw material residues containing astaxanthin obtained through the centrifugal separation in step (3) were mixed with a buffer solution of citric acid/sodium citrate with a concentration of 0.1 mol/L and a pH of 6.0 at a material-to-liquid ratio of 1 g : 20 mL to obtain a leachate, step (2) was repeated, and the re-leaching was repeated six times; and

(5) the raw material residues containing astaxanthin obtained through the centrifugal separation in step (4) were mixed with a buffer solution of citric acid/sodium citrate with a concentration of 0.1 mol/L and a pH of 5.0 at a material-to-liquid ratio of 1 g : 20 mL to obtain a leachate, step (2) was repeated, and the re-leaching was repeated four times.

Example 8

This example provides a method for preparing water-soluble astaxanthin complex. The method includes the following steps:

(1) Chlorella (wet algae) was taken out from a refrigerator of -20 °C and thawed, 10 g of Chlorella algae mud were weighed and added with 40 mL of a buffer solution of citric acid/sodium citrate with a concentration of 0.1 mol/L and a pH of 3.0, they were mixed thoroughly, and the mixed solution of Chlorella was disrupted for 60min with an ultrasonic disruptor to obtain a leachate;

(2) the leachate in step (1) was transferred to a 50 mL brown finger-shaped bottle, placed on a magnetic stirrer, and stirred and leached at 35 °C for 12 h in a closed environment without light; and

(3) the mixed solution after leaching in step (2) was transferred to a centrifuge tube and centrifuged at 3 °C and 7000 rpm for 60 min with a centrifuge, and a supernatant after centrifugation was filtered with a disposable filter with a pore size of 2.5 $\mu$m to obtain water-soluble astaxanthin complex that was uniform, stable, orange-red and translucent.

Example 9

This example provides a method for preparing water-soluble astaxanthin complex. The method includes the following steps:

(1) Rhodotorula fafu was taken out from a refrigerator of -20 °C and thawed, 0.5 g of Rhodotorula fafu mud were weighed and added with 10 mL of a buffer solution of malic acid/sodium malate with a concentration of 0.15 mol/L and a pH of 6.5, they were mixed thoroughly, and the mixed solution of Rhodotorula fafu was disrupted for 60 min with an ultrasonic disruptor to obtain a leachate;

(2) the leachate in step (1) was transferred to a 50 mL brown finger-shaped bottle, placed on a magnetic stirrer, and stirred and leached at 20 °C for 48 h in a closed environment without light; and

(3) the mixed solution after leaching in step (2) was transferred to a centrifuge tube and centrifuged at 5 °C and 9000 rpm for 40 min with a centrifuge, and a supernatant after centrifugation was filtered with a disposable filter with a pore size of 3 μm to obtain water-soluble astaxanthin complex that was uniform, stable, orange-red and translucent.

Example 10

This example provides a method for preparing water-soluble astaxanthin complex, which is the same as that in Example 1 except that "a buffer solution of citric acid/sodium citrate" in step (1) was replaced with "a buffer solution of tartaric acid/sodium tartrate".

Example 11

This example provides a method for preparing water-soluble astaxanthin complex, which is the same as that in Example 1 except that "a buffer solution of citric acid/sodium citrate" in step (1) was replaced with "a solution of citric acid".

Example 12

This example provides a method for preparing water-soluble astaxanthin complex, which is the same as that in Example 7 except that 0.001 g of ATP synthase β-subunit were added in last four re-extractions in step (5).

II. Test and Result

1. Extraction rate

The content of astaxanthin in a solution of water-soluble astaxanthin complex is tested by the following method: astaxanthin is extracted from water-soluble astaxanthin complex by use of methanol and chloroform. 1 mL of water-soluble astaxanthin complex was extracted with organic solvents and 1 mL of methanol and 2 mL of chloroform were added in sequence. Water-soluble astaxanthin complex was extracted until an aqueous phase was almost colorless, and organic phases were collected, dried with nitrogen, and passed through an organic filter membrane of 0.22 μm by use of 0.5 mL of methanol and 0.5 mL of methyl t-butyl ether (1/1, v/v) to collect samples for a later analysis. Standard astaxanthin (1 mg) was fully dissolved in 5 mL of methanol and 5 mL of methyl t-butyl ether (1/1, v/v) and passed through an organic filter membrane of 0.22 μm to collect samples for a later analysis. The content of astaxanthin was determined by HPLC.

Chromatographic column: YMC-Carotenoid-C30 chromatographic column (4.6 mm × 250 mm, 5 μm); mobile phase: A is methanol and B is methyl t-butyl ether; linear gradient elution: B is 10% from 0 to 15 min, B increases from 10% to 60% from 15 to 25 min, and B decreases from 60% back to 10% from 25 to 35 min; flow rate: 1 mL/min; DAD detection wavelength: 476nm; column oven temperature: 35 °C; sample volume: 20 μL. The content of astaxanthin in 1 mL of water-soluble astaxanthin complex is calculated according to formula (1):

$$\mu_g = \frac{S_1 \times m \times A}{S_2} \quad (1)$$

In formula (1), $\mu_g$ denotes the mass of astaxanthin in 1 mL of water-soluble astaxanthin complex, $S_1$ denotes the peak area of astaxanthin in water-soluble astaxanthin complex, $S_2$ denotes the peak area of standard astaxanthin, m denotes the mass of astaxanthin in 20 μL of standard solution of astaxanthin and is 2 mg, and A denotes a conversion factor and is 50.

The extraction rate is calculated as follows: all astaxanthin is extracted from a raw material containing astaxanthin according to the above experimental method, the total content of astaxanthin in the raw material is obtained, and the extraction rate of astaxanthin is calculated according to formula (2):

$$\text{Extraction rate } \% = \frac{\text{Content of astaxanthin in water−soluble astaxanthin}}{\text{Total content of astaxanthin in the raw material containing astaxanthin}} \times 100\% \quad (2)$$

The extraction rates of astaxanthin in Examples 1 to 5 are calculated according to the above method and shown in Table 1.

Table 1

| Example | Example 1 | Example 2 | Example 3 | Example 4 | Example 5 |
|---|---|---|---|---|---|
| Extraction Rate (wt %) | 37.45±0.87 | 3.93±0.34 | 14.17±1.22 | 24.13±1.41 | 1.44±0.25 |

The extraction rates of six re-extractions in Example 6 are calculated according to the above method. The results of the first extraction in Example 1 and the six re-extractions in Example 6 are shown in Table 2.

Table 2

| Extraction Times | Extraction Rate (wt %) |
|---|---|
| First extraction | 37.45±0.87 |
| First re-extraction | 11.66±0.98 |
| Second re-extraction | 8.11±0.55 |
| Third re-extraction | 3.71±1.29 |
| Fourth re-extraction | 1.89±0.49 |
| Fifth re-extraction | 0.52±0.22 |
| Sixth re-extraction | 0.54±0.17 |

The extraction rates of the last four re-extractions in Example 7 are calculated according to the above method. The results of the re-extractions are shown in Table 3.

Table 3

| Extraction Times | Example 7 Extraction Rate (wt %) |
|---|---|
| Seventh re-extraction | 1.67±0.44 |
| Eighth re-extraction | 1.31±0.37 |
| Ninth re-extraction | 0.61±0.24 |
| Tenth re-extraction | 0.58±0.11 |

2. Antioxidant property

Method for analyzing the antioxidant property: an antioxidant capacity of water-soluble astaxanthin complex is determined through the scavenging rate of ABTS$^+$ free radicals. A solution of potassium persulfate with a concentration of 2.6 mmol/L and a solution of ABTS with a concentration of 7.4 mmol/L were mixed in a brown finger-shaped bottle in equal amounts and reacted at room temperature for 12 h without light to obtain an operating solution. The overnight ABTS$^+$ solution was diluted with absolute ethanol until the absorbance at 734 nm was 0.700±0.20 for use.

10 μL of water-soluble astaxanthin complex were placed in a 96-well plate and added with 200 μL of diluted ABTS$^+$ solution. The mixed solution was mixed thoroughly and shaken uniformly and reacted for 1 h at normal temperature with no light. A microplate reader was used for measuring the absorbance at a wavelength of 734 nm and the absorbance was recorded as *A1*.

10 µL of water-soluble astaxanthin complex were mixed thoroughly with 200 µL of absolute ethanol and reacted for 1 h at normal temperature with no light. The microplate reader was used for measuring the absorbance at a wavelength of 734 nm and the absorbance was recorded as *A2*.

10 µL of absolute ethanol were mixed thoroughly with 200 µL of ABTS$^+$ solution and reacted for 1 h at normal temperature with no light. The microplate reader was used for measuring the absorbance at a wavelength of 734 nm and the absorbance was recorded as *A0*.

The above steps were repeated three times and data was recorded respectively. The scavenging rate of ABTS$^+$ free radicals by water-soluble astaxanthin complex is calculated according to formula (3):

$$\text{Scavenging rate } \% = \frac{A_0 - (A_1 - A_2)}{A_0} \times 100\% \quad (3)$$

In formula (3), *A1* denotes the absorbance after the sample reacts with ABTS$^+$ free radicals, *A2* denotes the absorbance of the sample itself, *and A0* denotes the absorbance of the blank group.

The antioxidant property of water-soluble astaxanthin complex in Examples 1 to 5 was calculated according to the above method and expressed as the scavenging rate of ABTS$^+$ free radicals. The results are shown in Table 4.

Table 4

| Example | Scavenging Rate (wt %) |
|---------|------------------------|
| Example 1 | 82.99±2.67 |
| Example 2 | 32.51±1.98 |
| Example 3 | 50.48±2.34 |
| Example 4 | 68.89±2.55 |
| Example 5 | 25.32±1.56 |

The following points can be seen from Table 1 and Table 4:

(1) It can be seen from Examples 1 to 4 that the effect of the buffer system of citric acid/sodium citrate in preparing water-soluble astaxanthin complex is related to the pH of the system. The extraction rate of astaxanthin increases with an increase of the pH within the pH range of 3.0 to 6.0, and the antioxidant property of the obtained water-soluble astaxanthin complex increases with an increase of the pH. When the pH is 5.0 to 6.0, the effect in preparing water-soluble astaxanthin complex is better, the highest extraction rate of astaxanthin can reach 37.45 %, and the antioxidant property can be up to 82.99 wt% in terms of the scavenging rate of ABTS$^+$ free radicals.

(2) It can be seen from Examples 1 and 5 that the buffer system of citric acid/sodium citrate is used in Example 1, and the buffer solution of glycine/HCl is used in Example 5; in Example 1, the extraction rate is 37.45 wt%, and the scavenging rate of ABTS$^+$ free radicals is 82.99 wt%, while in Example 5, the extraction rate is only 1.44 wt% and the scavenging rate of ABTS$^+$ free radicals is only 25.32 wt%, which shows that the present application improves the extraction rate and antioxidant property of water-soluble astaxanthin complex by selecting a particular buffer solution system.

It can be seen from Table 2 and Table 3 that the raw material residues of astaxanthin after leaching and extraction still contain astaxanthin and can be extracted multiple times to improve the overall extraction rate of the process, and the extraction rate of astaxanthin gradually decreases when the same pH buffer solution is used for leaching and extraction multiple times and can be improved again by changing the ratio or pH of the pH buffer solution. Due to mild conditions and simple operations of leaching and extraction, the extraction rate can be improved through multiple times of leaching and extraction, which is very easy for industrial production. The overall extraction rate reaches 68.05 wt% after 10 re-extractions in Example 7, which has a high industrial application value.

Moreover, Examples 8 and 9 can achieve similar extraction effects to those of Example 1 and can also prepare water-soluble astaxanthin complex in one step. Details are not repeated here.

Example 10 that uses a buffer solution of tartaric acid/sodium tartrate has a lower extraction rate and a poorer antioxidant property of the prepared water-soluble astaxanthin complex than Example 1 that uses a buffer solution of citric acid/sodium citrate. In the present application, the astaxanthin extraction effect is intermediate and a relatively good extraction rate and antioxidant property can be achieved when a system of malic acid/sodium malate is used.

In Example 11, it is difficult to ensure the stability of the pH in the extraction process when a solution of citric acid is used instead of a pH buffer system. Relative to the use of a buffer solution of citric acid/sodium citrate buffer, the extraction rate decreases.

In Example 12, proteins are added in the re-extraction process so that the assembly of astaxanthin in the solution is promoted and the extraction rate of astaxanthin is improved.

In summary, the method for preparing water-soluble astaxanthin complex provided by the present application includes mixing a raw material containing astaxanthin with a solution containing an organic acid and performing cell disruption and leaching and can enable natural astaxanthin to interact with components such as proteins, nucleic acids, or polysaccharides that naturally exist in the raw material, where the extraction rate of the first extraction is 1.44wt % or more and can be 37.45 wt% or more at most and the overall extraction rate of multiple extractions can reach 68.05 wt%. The method can obtain water-soluble astaxanthin complex in one step without solvent residues, requires a natural source of astaxanthin, and is simple to operate and easy to industrialize.

The applicant has stated that although the detailed structure characteristics of the present application are described through the examples described above, the present application is not limited to the detailed structure characteristics described above, which means that the implementation of the present application does not necessarily depend on the detailed structure characteristics described above. It should be apparent to those skilled in the art that any improvements made to the present application, equivalent replacements of units selected in the present application and addition of auxiliary units thereof, and selections of specific methods, etc., all fall within the protection scope and the disclosed scope of the present application.

**Claims**

1.  A method for preparing water-soluble astaxanthin complex, comprising:

    (1) mixing a raw material containing astaxanthin with a solution containing an organic acid and performing cell disruption to obtain a leachate; and
    (2) stirring and leaching the leachate in step (1) and performing solid-liquid separation to obtain water-soluble astaxanthin complex.

2.  The method according to claim 1, wherein the raw material containing astaxanthin comprises any one selected from the group consisting of Haematococcus pluvialis, Phaffia rhodozyma, crustaceans, chlorella, and a combination of at least two selected therefrom, preferably, Haematococcus pluvialis.

3.  The method according to claim 1 or 2, wherein the organic acid in the solution containing an organic acid in step (1) has a mass concentration of 0.1 to 2.0 moL/L.

4.  The method according to any one of claims 1 to 3, wherein the solution containing an organic acid has a pH of 3.0 to 6.5, preferably 5.0 to 6.3.

5.  The method according to any one of claims 1 to 4, wherein the organic acid comprises any one selected from the group consisting of malic acid, tartaric acid, glycine, oxalic acid, citric acid, and a combination of at least two selected therefrom, preferably, citric acid.

6.  The method according to any one of claims 1 to 5, wherein the solution containing an organic acid is a pH buffer solution of an organic acid.

7.  The method according to claim 6, wherein the pH buffer solution of an organic acid comprises any one selected from the group consisting of a buffer solution of malic acid and sodium malate, a buffer solution of tartaric acid and sodium tartrate, a buffer solution of glycine and HCl, a buffer solution of oxalic acid and sodium oxalate, a buffer solution of citric acid and sodium citrate, and a combination of at least two selected therefrom, preferably, a buffer solution of citric acid and sodium citrate.

8.  The method according to any one of claims 1 to 7, wherein a material-to-liquid ratio of the raw material containing astaxanthin to the solution containing an organic acid is 0.1-10 g: 10-40 mL.

9. The method according to any one of claims 1 to 8, wherein in step (1) and/or step (2), an auxiliary agent is added to assist in leaching;

preferably, the auxiliary agent comprises any one selected from the group consisting of proteins, nucleic acids, polysaccharides, and a combination of at least two selected therefrom.

10. The method according to any one of claims 1 to 9, wherein the stirring and leaching in step (2) is performed for 6 to 72 hours, preferably, 12 to 48 hours;

preferably, the stirring and leaching is performed at a temperature of 20 to 35 °C;
preferably, the stirring and leaching is performed under a closed condition without light.

11. The method according to any one of claims 1 to 10, wherein the solid-liquid separation in step (2) comprises centrifugal separation;

preferably, the centrifugal separation is performed at a temperature of 2 to 5 °C;
preferably, after the centrifugal separation, a supernatant obtained through the centrifugal separation is filtered with a filter to obtain a solution of water-soluble astaxanthin complex.

12. The method according to any one of claims 1 to 11, further comprising: (3) re-leaching raw material residues containing astaxanthin obtained through the solid-liquid separation in step (2) at least once;

preferably, the re-leaching comprises: mixing the raw material residues containing astaxanthin with a solution containing an organic acid to obtain a leachate and repeating step (2);
preferably, the re-leaching adopts the same solution containing an organic acid as step (1) and/or a different solution containing an organic acid from step (1);
preferably, the re-leaching adopts the same solution containing an organic acid as step (1) and then a different solution containing an organic acid from step (1).

13. The method according to any one of claims 1 to 12, comprising:

(1) mixing a raw material containing astaxanthin with a solution containing an organic acid and performing cell disruption to obtain a leachate, wherein the organic acid has a mass concentration of 0.1 to 2.0 moL/L, the solution containing an organic acid is a pH buffer solution of an organic acid with a pH of 3.0 to 6.5, and a material-to-liquid ratio of the raw material containing astaxanthin to the solution containing an organic acid is 0.1-10 g: 10-40 mL;
(2) stirring and leaching the leachate in step (1) for 6 to 72 h at 20 to 35 °C under a closed condition without light, performing centrifugal separation for 30 to 60 min at 2 to 5 °C and 7000 to 9000 r/min, and filtering a supernatant obtained through the centrifugal separation with a filter to obtain water-soluble astaxanthin complex; and
(3) mixing raw material residues containing astaxanthin obtained through solid-liquid separation in step (2) with a solution containing an organic acid to obtain a leachate and repeating step (2), wherein the re-leaching is performed at least once.

14. An aqueous solution of astaxanthin prepared by the method for preparing water-soluble astaxanthin complex according to any one of claims 1 to 13.

15. A lyophillized powder of astaxanthin prepared by lyophillizing the aqueous solution of astaxanthin according to claim 14.

Solution containing an organic acid

```
┌──────────┐      ┌────────┐   ┌──────────┐   ┌──────────┐   ┌──────────┐   ┌──────────────────┐
│Raw material│     │        │   │          │   │Stirring and│  │Solid-liquid│ │Water-soluble astaxanthin│
│containing │ ───→ │ Mixing │──→│Cell disruption│→│ leaching │─→│separation│─→│      complex     │
│astaxanthin│      │        │   │          │   │          │   │          │   └──────────────────┘
└──────────┘      └────────┘   └──────────┘   └──────────┘   └──────────┘   ┌──────────────────┐
                                                                            │Raw material residues│
                                                                            │containing astaxanthin│
                                                                            └──────────────────┘
```

Solution containing an organic acid

1/1

Fig. 1

Fig. 2

## INTERNATIONAL SEARCH REPORT

| International application No. |
|---|
| **PCT/CN2020/120622** |

**A. CLASSIFICATION OF SUBJECT MATTER**

C07C 403/24(2006.01)i; A61K 31/122(2006.01)i; A61P 39/06(2006.01)i; A23L 33/105(2016.01)i; A23L 33/145(2016.01)i

According to International Patent Classification (IPC) or to both national classification and IPC

**B. FIELDS SEARCHED**

Minimum documentation searched (classification system followed by classification symbols)

C07C A61K A61P A23L

Documentation searched other than minimum documentation to the extent that such documents are included in the fields searched

Electronic data base consulted during the international search (name of data base and, where practicable, search terms used)

CNABS, CNTXT, VEN, USTXT, EPTXT, WOTXT, CNKI, 万方, Web of Science, 百度学术, Registry, Caplus, 中国海洋大学, 齐祥明, 梁康琳, 黄文灿, 毛相朝, 虾青素, 提取, 分离, 萃取, 水溶性, 稳定性, 纳米复合物, 有机酸, 苹果酸, 酒石酸, 甘氨酸, 草酸, 柠檬酸, 雨生红球藻, 法夫红酵母, 小球藻, Astaxanthin, water solubility, stability, nano-complex, organic acid, malic acid, tartaric acid, glycine, extract+, Haematococcus pluvialis, Rhodotorula fafu, 472-61-7/RN

**C. DOCUMENTS CONSIDERED TO BE RELEVANT**

| Category* | Citation of document, with indication, where appropriate, of the relevant passages | Relevant to claim No. |
|---|---|---|
| A | CN 109419819 A (OCEAN UNIVERSITY OF CHINA) 05 March 2019 (2019-03-05) entire document, in particular paragraphs 007-011 | 1-15 |
| A | CN 104905321 A (TIANJIN INSTITUTE OF INDUSTRIAL BIOTECHNOLOGY, CHINESE ACADEMY OF SCIENCES et al.) 16 September 2015 (2015-09-16) entire document, in particular paragraphs 008-013 | 1-15 |
| A | CN 105483009 A (CHENGUANG BIOTECH GROUP CO., LTD.) 13 April 2016 (2016-04-13) entire document, in particular embodiments 3 and 4 | 1-15 |
| A | CN 107011225 A (STATE DEVELOPMENT & INVESTMENT CORPORATION et al.) 04 August 2017 (2017-08-04) entire document, in particular paragraphs 008-014 | 1-15 |

☑ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **16 April 2021** | **28 April 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2020/120622** |

**C.    DOCUMENTS CONSIDERED TO BE RELEVANT**

| Category* | Citation of document, with indication, where appropriate, of the relevant passages | Relevant to claim No. |
| --- | --- | --- |
| A | 倪辉 等 (NI, Hui et al.). "酸法破壁条件对法夫酵母虾青素提取效果的影响 (Effects of Disrupting Conditions on Extracting Astaxanthin from Phaffia rhodozyma by Acid Method)" 农业工程学报 (*Transactions of the Chinese Society of Agricultural Engineering*), Vol. 21, No. 3, 31 March 2005 (2005-03-31), ISSN: 1002-6819,<br>    pp. 176-180, in particular p. 177, section 2.1 | 1-15 |
| A | Ravit Edelmana et al. "Potato protein-based carriers for enhancing bioavailability of astaxanthin" *Food Hydrocolloids*, Vol. 96, 29 April 2019 (2019-04-29), ISSN: 0268-005X,<br>    pages 72-80, in particular page 73, right-hand column | 1-15 |
| A | Chengzhen Liu et al. "Fabrication and Characterization of β-Lactoglobulin-Based Nanocomplexes Composed of Chitosan Oligosaccharides as Vehicles for Delivery of Astaxanthin" *Journal of Agricultural and Food Chemistry*, Vol. 66, 08 June 2018 (2018-06-08), ISSN: 0021-8561,<br>    pages 6717-6726, in particular page 6718 right-hand column paragraph 2 | 1-15 |

Form PCT/ISA/210 (second sheet) (January 2015)

**INTERNATIONAL SEARCH REPORT**
Information on patent family members

International application No.

**PCT/CN2020/120622**

| Patent document cited in search report | | | Publication date (day/month/year) | Patent family member(s) | | | Publication date (day/month/year) |
|---|---|---|---|---|---|---|---|
| CN | 109419819 | A | 05 March 2019 | None | | | |
| CN | 104905321 | A | 16 September 2015 | None | | | |
| CN | 105483009 | A | 13 April 2016 | CN | 105483009 | B | 21 February 2020 |
| CN | 107011225 | A | 04 August 2017 | CN | 107011225 | B | 13 August 2019 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 110724083 A
- CN 109608376 A
- CN 107805215 B
- CN 108403666 A
- CN 109646425 A

**Non-patent literature cited in the description**

- **WANG LINGZHAO et al.** STUDY ON THE TECHNOLOGY OF EXTRACTING ASTAXANTHIN FROM HAEMATOCOCCUS PLUVIALIS BY MICROWAVE METHOD. *Food Research and Development,* vol. 28 (12), 96-100

- **NAVIDEH A et al.** Effects of Selected Polysorbate and Sucrose Ester Emulsifiers on the Physicochemical Properties of Astaxanthin Nanodispersions. *Molecules,* (18), 768-777
- **XIAOLIN CHEN et al.** The preparation and stability of the inclusion complex of astaxanthin with β-cyclodextrin. *Food Chemistry,* (101), 1580-1584